# EUROPEAN PATENT SPECIFICATION

(11) **EP 2 720 673 B1**
(45) Date of publication and mention of the grant of the patent: **26.12.2018**
(21) Application number: 12728755.5
(22) Date of filing: 15.06.2012
(51) Int. Cl.: A61K 8/49, A61K 8/97, A61Q 7/02, A61K 8/34, A61K 8/9783

(54) **COMPOSITIONS FOR REDUCING HUMAN HAIR GROWTH**
ZUSAMMENSETZUNGEN ZUR VERMINDERUNG DES MENSCHLICHEN HAARWACHSTUMS
COMPOSITIONS POUR LA REDUCTION DE LA POUSSE DES CHEVEUX HUMAINS

(30) Priority: 16.06.2011 GB 201110132
(43) Date of publication of application: 23.04.2014
(73) Proprietor: Reckitt & Colman (Overseas) Limited, Slough, Berkshire SL1 3UH (GB)
(72) Inventor: DODD, Karen, Hull HU8 7DS (GB); MCGOWAN, Pamela, Hull HU8 7DS (GB); TINDAL, Anne, Hull HU8 7DS (GB); WARWICK, Jane Margaret, Hull HU8 7DS (GB)
(74) Representative: O'Brien, Niall James
(86) International application number: PCT/GB2012/051377
(87) International publication number: WO 2012/172358

(56) References cited:
- EP-A2- 1 074 240
- WO-A1-01/72266
- WO-A1-2005/067953
- FR-A1- 2 791 255
- FR-A1- 2 890 310
- KR-A- 20070 017 490
- US-A1- 2002 197 290
- "Phytoconstituents in Gymnema sylvestre", "Phytoconstituents and Pharmacological Properties of Gymnema sylvestre" , 2014, Retrieved from the Internet: URL:https://www.hindawi.com/journals/bmri/ 2014/830285/tab1/ [retrieved on 2014]

## Description

The invention relates to a cosmetic composition for modification of hair growth in humans, and methods of using said composition.

In humans, particularly women, visible body hair is considered unattractive in some cultures. Many and various procedures have been employed to remove unwanted hair, including shaving, electrolysis, application of depilatory creams or lotions which dissolve hair, waxing, plucking (manual or mechanical using an epilator), laser therapy and therapeutic antiandrogens, sometimes by injection. These procedures generally have drawbacks associated with them. Shaving may cause skin abrasion or cuts, and can lead a perception of roughness caused by sharp, stubbly hairs as the hair reappears after shaving. Electrolysis can be particularly effective at keeping a treated area free of hair for prolonged periods of time, but can be time consuming and painful. Electrolysis also carries the potential risk of scarring if excessive currents are applied.

Depilatory creams are effective, but the regime of their application must be carefully controlled to avoid risk of skin irritation. Waxing and plucking of hairs is an intrinsically painful and uncomfortable process. Antiandrogens, which maybe used to treat female hirsutism, may have unwanted side effects because of their effect on the hormone balance in the body.

Hence it would be desirable to provide a chemically or biochemically based method for the inhibition, reduction or delay of hair growth in humans.

It has previously been disclosed that the rate and character of hair growth can be altered by applying to the skin inhibitors of certain enzymes. These inhibitors include inhibitors of 5-alpha reductase, ornithine decarboxylase, S-adenosylmethionine decarboxylase, gamma- glutamyl transpeptidase, and transglutaminase. These chemicals function by modification of a biochemical pathway to inhibit the production of a final product, and so potentially lead to unwanted side effects in the user arising from the loss of specific amino acids.

EP1750736 describes uses for the plant extract of gymnestra sylvestris in compositions which influence hair growth in humans.

It is desirable to provide alternate methods for the inhibition, reduction or prevention of hair growth in mammals. It is also desirable to use chemical compounds which are derived from natural sources, in that such chemicals are more likely to be accepted by users as less harmful than synthetic materials.

In one aspect, the invention provides a depilatory composition comprising at least two different hair growth inhibition additives comprising a combination of from 0.01 to 5 wt% of the plant extract Gymnestra sylvestra and from 0.001 to 10 wt% chelidonine, wherein the weight ratio, based on active ingredient, of gymnestra sylvestra extract to chelidonine is between from 1:1 to 250:1.

In order to put the method of the invention into practice, the at least two hair growth inhibitors are suitably included in a topical composition along with a dermatologically or cosmetically acceptable vehicle or carrier. Suitably the vehicle or carrier is adapted to be spread upon the skin. Accordingly, the present invention also relates to topical compositions comprising a dermatologically and/or cosmetically acceptable carrier and at least two hair growth inhibitors in an amount effective to reduce hair growth.

Examples of suitable vehicles or carriers include acetone, alcohols, creams, lotions or gels. When the carrier is a cream or lotion, it is preferably in the form of an oil in water or a water in oil emulsion.

The composition may be a solid, semi-solid, or liquid. The composition may be a cosmetic or a therapeutic product in the form of an ointment, lotion, foam, cream, gel, or solution. The composition may also be in the form of a shaving preparation or an after-shave product intended for application to the skin after shaving or after waxing.

The hair growth inhibitors are suitably present in the composition at a level from 0.05% to 1%, and most preferably 0.05% to 0.1% based on active ingredient.

In a more preferred emdodiment the weight ratio, based on active ingredient, of gymnestra sylvestra extract to chelidonine is between from 10:1 to 50:1.

The hair growth inhibitors may be supplied undiluted, or supplied as a formulated premix with solvent(s) and/or water.

The vehicle or carrier for the at least two hair growth inhibitors can be inert or can possess cosmetic, physiological and/or pharmaceutical benefits of its own. Vehicles can be formulated with liquid or solid emollients, solvents, thickeners, humectants and/or powders. Emollients include cetyl alcohol, stearyl alcohol, triglyceride oils, oleyl alcohol, isopropyl laurate, polyethylene glycols, petroleum jelly, and esters such as myristyl myristate. Solvents include ethyl alcohol, isopropanol, acetone, diethylene glycol, ethylene glycol, propylene glycol, butylene glycol, hexylene glycol, 2-methyl-1,3-propanediol, dimethyl isosorbide, dimethyl sulfoxide, and dimethyl formamide.

The composition also can include components that enhance the penetration of the inhibitor into the skin and/or to the site of action. Examples of penetration enhancers include urea, cis-fatty acids (e.g., oleic acid, palmitoleic acid), acetone, laurocapram, dimethylsulfoxide, 2-pyrrolidone, oleyl alcohol, glyceryl-3 stearate, propan-2-ol, myristic acid isopropyl ester, cholesterol, and propylene glycol.

A penetration enhancer can be added suitably at concentrations of 0.1% to 20% or preferably 0.5% to 5% by weight. The composition also can be formulated to provide a reservoir within or on the surface of the skin to provide for a continual slow release of the at least two hair growth inhibitors. The composition also may be formulated to evaporate slowly from the skin while the at least two hair growth inhibitors slowly penetrates into the surface of the skin. In particular, one or more of the hair groth inhibitors in the composition may also be microencapsulated to enable slow release.

The composition should be topically applied to a selected area of the body from which it is described to reduce hair growth. For example, the composition can be applied to the face, particularly to the beard area of the face, i.e., the cheek, neck, upper lip, and chin. The composition also may be used as an adjunct to other methods of hair removal including shaving, waxing, mechanical epilation, chemical depilation, electrolysis and or laser-assisted hair removal as well as skin moisturing after hair removal.

The composition can also be applied to the legs, groin region (bikini area), arms, torso, face or armpits. The composition is particularly suitable for reducing the growth of unwanted hair in women having hirsutism or other conditions. In humans, the composition should be suitably applied once a day, preferably at least twice a day, to achieve a perceivable reduction in hair growth. Perception of reduced hair growth could occur as early as 24 hours or 48 hours (for instance, between normal shaving intervals) following use, but is typically in the order of two weeks. Reduction in hair growth is demonstrated when, for example, the rate of hair growth is slowed, the need for removal is reduced, the subject perceives less hair on the treated site, or quantitatively, when the weight of hair removed is reduced.

The composition may also suitably be applied as part of the hair removal routine, and where the composition will be in contact with the skin for a relatively short period of time, typically greater than 1 minute and less than 10 minutes.

The composition may also further be used as part of an everyday daily personal care routine, and be present in such products as a moisturing wipe, shower gel, deodorant, cream, lotion, balm, spray, aerosol, and cleansing lotions.

In alternative embodiments, the topically active therapeutic or cosmetic composition may be optionally combined with other ingredients such as moisturisers, foaming agents, cosmetic adjuvants, anti-oxidants, surfactants, foaming agents; conditioners, humectants, fragrances, viscosity modifiers, buffering agents, preservatives, and the like, in order to produce cosmetic or pharmaceutical products such as shaving creams, shaving gels, shaving powders, chemical depilatory creams and the like.

The short-term or instantaneous method of hair removal can be performed by any method well known in the art, including, but not limited to chemical or mechanical depilation such as, shaving, wax depilation, chemical depilation, mechanical plucking with an epilator and combinations thereof. By short-term or instantaneous it is meant that the hair removal method removes the hair by cutting, dissolution or plucking, but does not prevent or inhibit regrowth of the hair. Chemical depilation means the dissolution of hair by a depilation agent such as an alkaline thioglycolate.

### Examples (outside the scope of the invention)

The study used an *ex vivo* plucked human hair assay to examine the effect of a series of test items on outer root sheath (ORS) cell proliferation. The test items examined were Product B (contains Gymnema Sylvestre Leaf Extract) and Product C (contains highly purified plant molecule chelidonine).

### Results

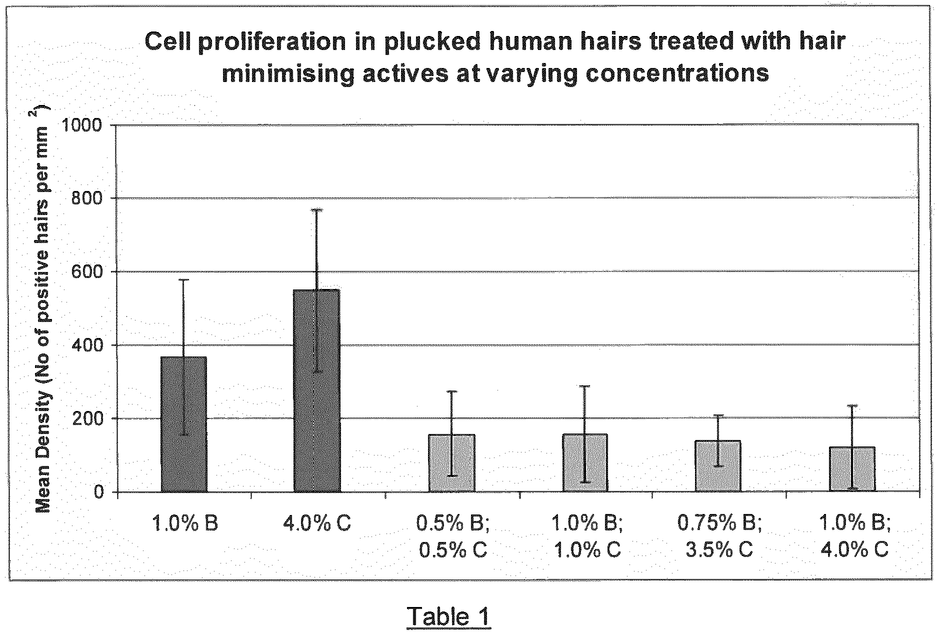

### Effect of test products on cell proliferation in plucked human hairs

Plucked human hairs were cultured *ex vivo* for 24 or 48 hours in proprietary cell culture media with either test items or vehicle (either 5% water or 10% ethanol solution [ethanol solution is 40% in water]). Upon harvest the number of proliferating cells was assessed by whole mount IHC labelling with the proliferation marker Ki67. The number of positively labelled cells in fresh hairs and 24 hour vehicle 1 (5% water) treated hairs was similar. When PLS100 and KLS100 were used in combination, a synergistic effect was observed with a dose dependent decrease in the number of proliferating cells.

Analysis of untreated hairs cultured for 48 hours showed that the number of proliferating cells was similar to that observed in fresh hair, indicating that hairs can be maintained in the proprietary media in a proliferative state for extended periods of time.

Ki67 labelling indicates that the proprietary culture media used in this study maintains hairs in a proliferative state similar to that of freshly plucked hairs for at least 48 hours. This then provides a suitable *ex vivo* system in which to assess the effect test items have on cell proliferation within the outer root sheath (ORS) of the hair.

This *ex vivo* hair culture model was used to analyse the effect of Product B and product C on their own, as well as combinations of Products B + C on cell proliferation. The results show that when Product B and Product C were used in combination a clear dose dependent synergistic effect was observed.

Further modifications and developments can be made without departing from the scope of the invention described herein.

## Claims

1. A depilatory composition comprising at least two different hair growth inhibition additives comprising a combination of from 0.01 to 5 wt% of the plant extract Gymnestra sylvestra and from 0.001 to 10 wt% chelidonine, wherein the weight ratio, based on active ingredient, of gymnestra sylvestra extract to chelidonine is between from 1:1 to 250:1.

2. A cosmetic non-therapeutic method for reducing human hair growth comprising selecting an area of skin from which reduced hair growth is desired and applying to the area of skin a cosmetically acceptable composition according to claim 1.

3. The cosmetic non-therapeutic method of claim 2 wherein the composition is applied to the area of skin in combination with mechanical or chemical depilation.

## Patentansprüche

1. Haarentfernungszusammensetzung, umfassend mindestens zwei verschiedene haarwachstumshemmende Additive, umfassend eine Kombination von 0,01 bis 5 Gew.-% des Pflanzenextrakts Gymnestra sylvestra und 0,001 bis 10 Gew.-% Chelidonin, wobei das Gewichtsverhältnis, bezogen auf Wirkstoff, von Gymnestra-sylvestra-Extrakt zu Chelidonin 1:1 bis 250:1 beträgt.

2. Kosmetisches nichttherapeutisches Verfahren zur Verringerung des Wachstums von menschlichem Haar, bei dem man einen Hautbereich auswählt, von dem verringertes Haarwachstum gewünscht ist, und auf den Hautbereich eine kosmetisch unbedenkliche Zusammensetzung nach Anspruch 1 aufbringt.

3. Kosmetisches nichttherapeutisches Verfahren nach Anspruch 2, bei dem man die Zusammensetzung in Kombination mit mechanischer oder chemischer Enthaarung auf den Hautbereich aufbringt.

## Revendications

1. Composition épilatoire, comprenant au moins deux additifs différents d'inhibition de la croissance des poils, comprenant une combinaison allant de 0,01 à 5% en poids de l'extrait végétal de *Gymnestra sylvestra* et de 0,001 à 10% en poids de chélidonine, où le rapport pondéral, sur la base des ingrédients actifs, de l'extrait de *Gymnestra sylvestra* à la chélidonine va de 1:1 à 250:1.

2. Méthode non thérapeutique cosmétique destinée à réduire la croissance des poils humains, comprenant la sélection d'une zone de peau à partir de laquelle on souhaite réduire la croissance des poils et l'application à la zone de peau d'une composition cosmétiquement acceptable selon la revendication 1.

3. Méthode non thérapeutique cosmétique selon la revendication 2, dans laquelle la composition est appliquée à la zone de peau en combinaison avec une épilation mécanique ou chimique.
